# EUROPEAN PATENT APPLICATION

(11) **EP 4 755 309 A1**
(43) Date of publication of application: **10.06.2026**
(21) Application number: 24217734.3
(22) Date of filing: 05.12.2024
(51) Int. Cl.: A61B 6/42, A61B 6/00, A61B 6/58

(54) **INTERFACING BETWEEN X-RAY DETECTOR AND X-RAY IMAGING SYSTEM**

(71) Applicant: Koninklijke Philips N.V., 5656 AE Eindhoven (NL)
(72) Inventor: GRASS, Michael, Eindhoven (NL); VOGTMEIER, Gereon, Eindhoven (NL); HELLE, Michael Günter, 5656AG Eindhoven (NL); VON BERG, Jens, Eindhoven (NL); WEIß, Steffen, Eindhoven (NL)
(74) Representative: Philips Intellectual Property & Standards

(57) **Abstract**

The present invention relates to a computer-implemented method for converting image data of an X-ray detector to a predefined data format of an X-ray control device. The method comprises receiving a request from the X-ray control device to provide an X-ray image, the request comprising a set of predefined image parameters of the X-ray image, sending a trigger signal to the X-ray detector to provide X-ray image data acquired with the X-ray detector, and receiving the X-ray image data acquired by the X-ray detector from the X-ray detector. The method comprises further processing the received X-ray image data, thereby generating a processed X-ray image comprising the predefined image parameters, and providing the processed X-ray image to the X-ray control device.

## Description

### FIELD OF THE INVENTION

The present invention relates to a computer-implemented method for converting image data of an X-ray detector to a predefined data format of an X-ray control device, a data processing apparatus, an interface unit, an X-ray imaging system, a computer program, and a computer-readable storage medium.

### BACKGROUND OF THE INVENTION

In the future, there will be an increasing number of options for X-ray detectors which can be built into a diagnostic X-ray system. This next generation of hardware like X-ray detectors needs to be integrated into X-ray imaging systems preferably without always re-designing the complete data acquisition and processing hardware/software of the imaging system. These new detectors, which may be, for example, a dual or triple layer X-ray system capable of generating spectral X-ray images from one single shot of X-rays, may be developed by the manufacturer of the X-ray imaging system, or they can be provided by a third-party vendor. In the latter case, proper interfaces for system integration are mandatory. However, seamless integration requires the interaction between the detector and, e.g., the X-ray tube or the high voltage generator for matching parameter settings and timing synchronization as well as calibration and processing software.

### SUMMARY OF THE INVENTION

The inventors of the present invention have developed a method and an interface providing connectability and compatibility of different kinds of detectors with an X-ray imaging system. It provides a method of transferring the tube acquisition parameters to the detector system as well as a data format transferring the multi-layer detector data as processed data to the hosting X-ray system.

It is an object of the present invention to provide an improved method for converting image data of an X-ray detector to a predefined data format of an X-ray control device, that solves at least a part of the problems of the state of the art.

The object of the present invention is solved by the subject matter of the independent claims, wherein further embodiments are incorporated in the dependent claims.

The described embodiments pertain to a computer-implemented method for converting image data of an X-ray detector to a predefined data format of an X-ray control device, a data processing apparatus, an interface unit, an X-ray imaging system, a computer program, and a computer-readable storage medium. The embodiments described further may be combined in any possible way. Synergistic effects may arise from different combinations in various ways of the embodiments described further although these combinations might not be described explicitly in detail.

Further on, it shall be noted that all embodiments of the present invention concerning a method might be carried out with the order of the steps as described, nevertheless this has not to be the only and essential order of the steps of the method. The herein presented methods can be carried out with another order of the disclosed steps without departing from the respective method, unless explicitly mentioned to the contrary hereinafter.

According to a first aspect of the invention, there is provided a computer-implemented method for converting image data of an X-ray detector to a predefined data format of an X-ray control device. The method comprises receiving a request from the X-ray control device to provide an X-ray image, the request comprising a set of predefined image parameters of the X-ray image, sending a trigger signal to the X-ray detector to provide X-ray image data acquired with the X-ray detector, and receiving the X-ray image data acquired by the X-ray detector from the X-ray detector. The method comprises further processing the received X-ray image data, thereby generating a processed X-ray image comprising the predefined image parameters, and providing the processed X-ray image to the X-ray control device.

Thus, a method is proposed for device-independent image data processing that allows integration of detectors into X-ray imaging systems for providing compatibility of various types of detectors with existing systems. With this method it is possible to convert data from multiple types of detectors from different vendors in different data formats to a standard imaging system data format.

The computer-implemented method can be performed by a separate processing unit in an interface box, which is communicationally connected to both the, or it can be implemented into the X-ray detector or the X-ray imaging system. However, it may be also performed in the cloud, e.g., by a computer connected via a network.

After receiving a request to provide an X-ray image from the X-ray imaging system of a corresponding X-ray control device, a trigger signal is sent to the connected X-ray detector to provide acquired X-ray image data or to acquire the X-ray image data and to provide them. The X-ray image data provided by the X-ray detector are in turn received from the X-ray detector. Further, according to the method, the received X-ray image data are processed in order to generate a processed X-ray image. The processed X-ray image is generated to be in accordance with predefined image parameters that are comprised in the request of the X-ray imaging system. Further, the processed X-ray image data are provided to the X-ray control device.

In an embodiment of the invention, the set of predefined image parameters comprises a predefined size of the X-ray image, and processing the received X-ray image data comprises interpolating the received X-ray image data to generate the processed X-ray image with the predefined size.

In an embodiment of the invention, the method comprises further determining a data format of the received X-ray image data, and processing the received X-ray image data comprises translating the determined data format of the received X-ray image data into a predefined data format.

In an embodiment of the invention, determining a data format of the received X-ray image data comprises comparing the received X-ray image data with a plurality of data formats from a database, receiving information indicating the data format of the received X-ray image data from the X-ray detector or from an operator of the X-ray control device, and/or using a trained artificial intelligence to determine the data format of the received X-ray image data.

In an embodiment of the invention, the method comprises further analyzing the received X-ray image data with respect to an indication of whether the received X-ray image data has been acquired by the X-ray detector using an anti-scatter grid, and in case an indication of an anti-scatter is detected, processing the received X-ray image data comprises the removing grid artefacts of the anti-scatter grid from the received X-ray image data, or in case an indication of no anti-scatter is detected, processing the received X-ray image data comprises performing scatter correction of the received X-ray image data.

In an embodiment of the invention, the method comprises further receiving imaging parameters of the X-ray control device, and processing the received X-ray image data comprises processing the received X-ray image data on the basis of the received imaging parameters of the X-ray control device.

In an embodiment of the invention, the imaging parameters comprise an energy setting and/or an energy spectrum of an X-ray tube connected to the X-ray control device.

In an embodiment of the invention, the X-ray detector is a spectral detector comprising a plurality of detection layers, the received X-ray image data comprises a plurality of sub-images corresponding to the plurality of detection layers, and processing the received X-ray image data comprises at least one of correcting at least one of the plurality of sub-images for absorption effects due to another one of the plurality of sub-images, performing spectral processing using an X-ray energy spectrum of an X-ray tube connected to the X-ray control device, and calculating a conventional X-ray image based on the plurality of sub-images.

In an embodiment of the invention, the X-ray detector is a flat panel detector configured to perform phase stepping, the received X-ray image data comprises a plurality of phase-stepping images, and processing the received X-ray image data comprises at least one of performing phase contrast processing using an X-ray energy spectrum of an X-ray tube connected to the X-ray control device and grid information provided by the X-ray control device, and calculatng a conventional X-ray image based on the plurality of phase-stepping images.

In an embodiment of the invention, the received X-ray image data comprises a plurality of temporal sub-images acquired by the X-ray detector in a temporal series, and processing the received X-ray image data comprises at least one of selecting at least one of the plurality of temporal sub-images, calculating an integral image from the plurality of temporal sub-images, calculating a registered integral image from the plurality of temporal sub-images, and selecting a sharpest image from the plurality of temporal sub-images.

According to another aspect of the invention, there is provided a data processing apparatus for carrying out the steps of the method according to any one of the preceding embodiments.

According to another aspect of the invention, there is provided an interface unit comprising the data processing apparatus of the preceding embodiment, the interface unit comprising a first interface configured to be communicationally connected to the X-ray control device, and a second interface configured to be communicationally connected to the X-ray detector.

According to another aspect of the invention, there is provided an X-ray imaging system comprising the interface unit of the preceding embodiment, an X-ray control device, and an X-ray detector.

According to another aspect of the invention, there is provided a computer program comprising instructions which, when the program is executed by a computer, cause the computer to carry out the steps of the method according to any one of the preceding embodiments.

According to another aspect of the invention, there is provided a computer-readable storage medium comprising instructions which, when executed by a computer, cause the computer to carry out the steps of the method according to any one of the preceding embodiments.
Thus, the benefits provided by any of the above aspects equally apply to all of the other aspects and vice versa.

In summary, the invention relates to a computer-implemented method for converting image data of an X-ray detector to a predefined data format of an X-ray control device. The method comprises receiving a request from the X-ray control device to provide an X-ray image, the request comprising a set of predefined image parameters of the X-ray image, sending a trigger signal to the X-ray detector to provide X-ray image data acquired with the X-ray detector, and receiving the X-ray image data acquired by the X-ray detector from the X-ray detector. The method comprises further processing the received X-ray image data, thereby generating a processed X-ray image comprising the predefined image parameters, and providing the processed X-ray image to the X-ray control device.

One of the advantages of embodiments of the present invention is that different types of detectors can be directly connected to the imaging system without extra effort of data handling. A device and a method for detector-independent "raw-to-imaging-system-compatible" image processing unit that can be integrated in OEM detectors and/or as a stand-alone interface is provided with the functionalities of converting data from multiple types of detectors from different vendors in different data formats to a standard imaging system data format. Special additional data information of detectors can be mapped into a standard data container, which allows to make the X-ray system upgrade ready for the future.

These advantages are non-limiting and other advantages may be envisioned within the context of the present application.

The above aspects and embodiments will become apparent from and be elucidated with reference to the exemplary embodiments described hereinafter. Exemplary embodiments of the invention will be described in the following with reference to the following drawings:

### BRIEF DESCRIPTION OF THE DRAWINGS

Fig. 1 shows a block diagram of a computer-implemented method for converting image data of an X-ray detector to a predefined data format of an X-ray control device according to an embodiment of the invention.
Fig. 2 shows a schematic setup of an X-ray imaging system according to an embodiment of the invention.
Fig. 3 shows a block diagram with data flow and interfaces of a computer-implemented method for converting image data of an X-ray detector to a predefined data format of an X-ray control device according to another embodiment of the invention.
Fig. 4 shows a block diagram with data flow and interfaces of a computer-implemented method for converting image data of an X-ray detector to a predefined data format of an X-ray control device according to another embodiment of the invention.

### DETAILED DESCRIPTION OF EMBODIMENTS

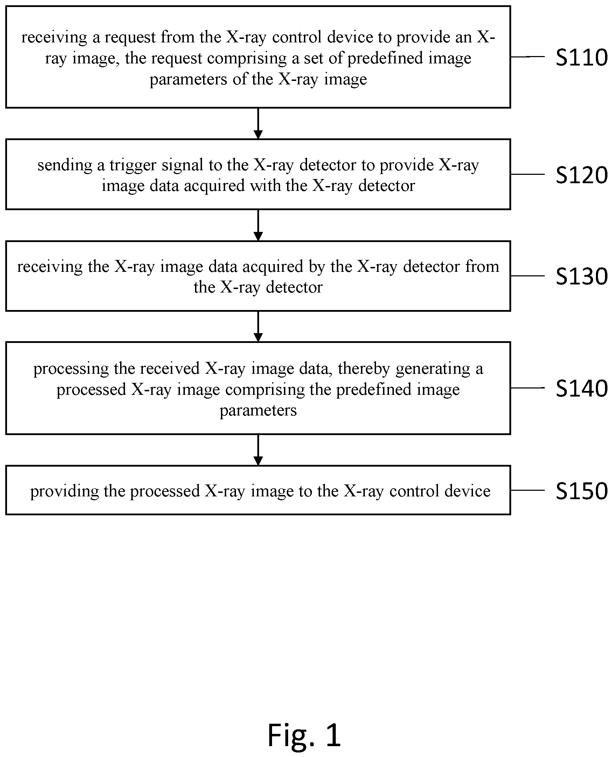

Fig. 1 shows a block diagram of a computer-implemented method for converting image data of an X-ray detector 140 to a predefined data format of an X-ray control device 130 according to an embodiment of the invention. The method comprises step S110 of receiving a request 170 from the X-ray control device 130 to provide an X-ray image, the request 170 comprising a set of predefined image parameters of the X-ray image, and step S 120 of sending a trigger signal 180 to the X-ray detector 140 to provide X-ray image data 150 acquired with the X-ray detector. The method comprises further step S130 of receiving the X-ray image data 150 acquired by the X-ray detector from the X-ray detector 140, step S140 of processing the received X-ray image data 150, thereby generating a processed X-ray image 160 comprising the predefined image parameters, and step S150 of providing the processed X-ray image 160 to the X-ray control device 130.

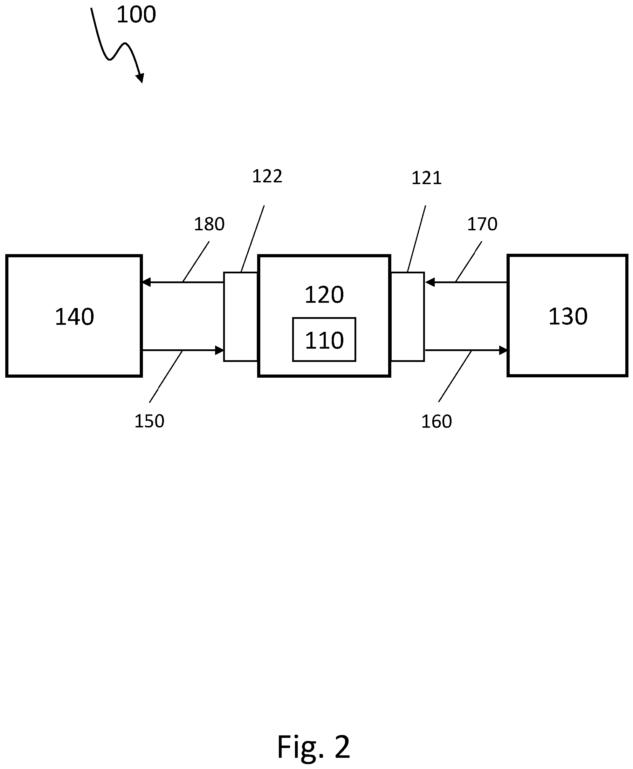

Fig. 2 shows a schematic setup of an X-ray imaging system 100 according to an embodiment of the invention. The X-ray imaging system comprises an X-ray control device 130, an X-ray detector 140, and an interface unit 120 including a data processing apparatus 110 for carrying out the steps of the method as described above. The interface unit is via a first interface 121 communicationally connected to the X-ray control device 130, and via a second interface 122 communicationally connected to the X-ray detector 130. When receiving a request 170 from the X-ray control device 130 to provide an X-ray image with a set of predefined image parameters of the X-ray image, a trigger signal 180 is sent to the X-ray detector 140 to provide X-ray image data 150 acquired with the X-ray detector. The provided X-ray image data 150 that are acquired by the X-ray detector are received from the X-ray detector 140 at the interface unit via the second interface 122. The data processing apparatus 110 processes the received X-ray image data 150 and generates a processed X-ray image 160 comprising the predefined image parameters. The processed X-ray image 160 is provided to the X-ray control device 130 via the first interface 121.

Many different types of detectors from different companies will be available on the market and it would be an advantage to be able to use these detectors but still be compatible with the existing system in internal data processing. This includes detectors with additional functionality like spectral detection, different detection principles including photon counting, or dark field x-ray imaging, but also different sizes and spatial resolution as well as acquisition modes and optimized selected field of view acquisition as well as processing. It may also include the simultaneous use of detectors from different vendors in a multi-layer system. Due to different OEM data formats and data definition a data conversion to existing interfaces in a system is necessary and will be more important to be future proof and upgrade compatible - also for refurbished systems.

Thus, main elements of an X-ray imaging system according to the invention are the interfaces between an X-ray system with tube, system control, and processing unit and a third-party spectral detector. It may include hardware definition and software functionality to compile data into a compatible format. To perform spectral X-ray imaging at the detector there are at least two potential ways to interface to this detector via a data container: As a first example, for spectral detectors with two or three multi-layer flat panel detectors after each other, the readout data from the detectors can be sent to the X-ray system and do all spectral processing at system side; in this case it is only the number of detector readout layers and their ordering which we need to transfer. As a second example, the detector may have processing intelligence by itself like an integrated processor; in this case a conventional photo and scatter image (or images based on different basis functions) and eventually even a noise estimate can be generated generate on the detector side, and these images can be sent to the X-ray system for further processing and visualization. For accurate processing a data protocol describing the acquisition needs to be send to the detector including HV settings, like kV, mA, pulse duration, and/or calibration data of the spectrum.

The same could be done for phase contrast imaging where either the different stepping images are transferred or the processed images like conventional, phase, and dark field are transferred in case the detector has processing capability by itself. The data container can be a modified DICOM container with private tags describing the content of the multiple images.

A module may be included to describe the complete data format, i.e., that the image comprises a number of sub-images with defined pixel size, pixel number, dynamic range and system setting parameters incl. timing. Information about raw data and how it can be compiled to standard compatible images can be given.

A modular concept with a key module of data conversion and additional modules to interface the physical elements/electrical signals using standard communication protocols like CAN, TCIP, etc., can be used. As far as new hardware physical interfaces would be required the stand-alone interface could provide different modules, which might be also used in a OEM detector with an integrated interface unit.

Thus, not just a passive data format conversion is provided, but an image processing image content conversion with synchronized parameter extraction for system control and system function logging adapted to the target imaging system.

For example, a complex (spectral) detector either must provide information by this interface unit how to generate a standard image from its different layers or provide this image by its own computing power. The same holds for providing scatter images to be used in scatter reduction software. It is up to the detector to define how these are being built based on its data. Detectors based on different technologies may come up with different approaches for this.
The simultaneous use of detectors from different vendors in a multi-layer system adds the need to compensate for the absorption and scattering of X-rays in more proximal detectors including their sensing circuitry when detecting with more distal detectors. Therefore, the data interface is proposed to contain data, optionally spectrally resolved data, for such absorption to be compensated in image reconstruction.

With reference to Fig. 2, the interface unit 120 can be connected to different types of X-ray detector 140 in order to transfer the X-ray image data 150 in an extended detector data format from the X-ray detector 140 to the X-ray control device 130 in a standard flat panel data format.

In a first example, the X-ray detector may be a standard flat panel detector. In this case, the request 170 may comprise requested image size and image processing like scatter correction, for example. After sending the trigger signal 180 and receiving the X-ray image data 150, the processing performed by the interface unit can comprise the detection of an anti-scatter grid. If an anti-scatter grid is detected, grid artefacts can be removed, and in case no anti-scatter grid is detected, scatter correction can be performed via software. Further, the X-ray image can be interpolated to the requested standard image size of the X-ray control device 130. The processed X-ray image 160 generated by the interface unit 120 is sent to the X-ray control device 130 for visualization and/or further processing. The interface unit can be either part of the detector, part of the DXR system, cloud based, or a separate box, for example, which can be physically connected.

In a second example, the X-ray detector may be a spectral multi-layer flat panel detector. In this case, the request 170 may comprise requested image size and imaging parameters like X-ray energy, and the X-ray tube energy spectrum, for example. After sending the trigger signal 180 and receiving the X-ray image data 150 which in this example can comprise a plurality of spectral X-ray images, the processing performed by the interface unit can comprise the detection of a number of sub images. The layer images can be corrected for absorption by more proximal layers, and a conventional image can be calculated. Spectral processing can be performed, like analytic, LUT, or AI, using the given tube energy spectrum information. Further, the X-ray image can be interpolated to the requested standard image size of the X-ray control device 130. The processed X-ray image 160 generated by the interface unit 120 is then sent to the X-ray control device 130 for visualization and/or further processing.

In a third example, the X-ray detector may be a flat panel detector with phase stepping. In this case, the request 170 may comprise requested image size and imaging parameters like X-ray energy, X-ray tube spectrum and G0 existence and characteristics. After sending the trigger signal 180 and receiving the X-ray image data 150, which in this example can comprise a plurality of phase stepping X-ray images, the processing performed by the interface unit can comprise the detection of a number of sub-images. A conventional image can be calculated, and phase contrast processing using analytic methods, LUT, or AI can be performed using the given tube spectrum and grid information. Further, the X-ray image can be interpolated to the requested standard image size of the X-ray control device 130. The processed X-ray image 160 generated by the interface unit 120 is sent to the X-ray control device 130 for visualization and/or further processing.

A standard image data format used in these examples for transferring processed image data between the interface unit and the X-ray control device may comprise a multi-layer matrix, with a first matrix layer representing a conventional X-ray image in requested matrix size. In case of spectral imaging, a second matrix layer may represent a photo image in requested matrix size, and a third matrix layer may represent a Compton image in standard matrix size. In case of phase contrast imaging, a second matrix layer may represent a phase image in standard matrix size, and a third matrix layer may represent a dark field image in standard matrix size. In contrast to that, all of the two or three detectors layers do provide information with different energy characteristic. Based on the information of these two or three images as received from the X-ray detector as X-ray image data, photo and scatter images or material images can be generated by decomposition.

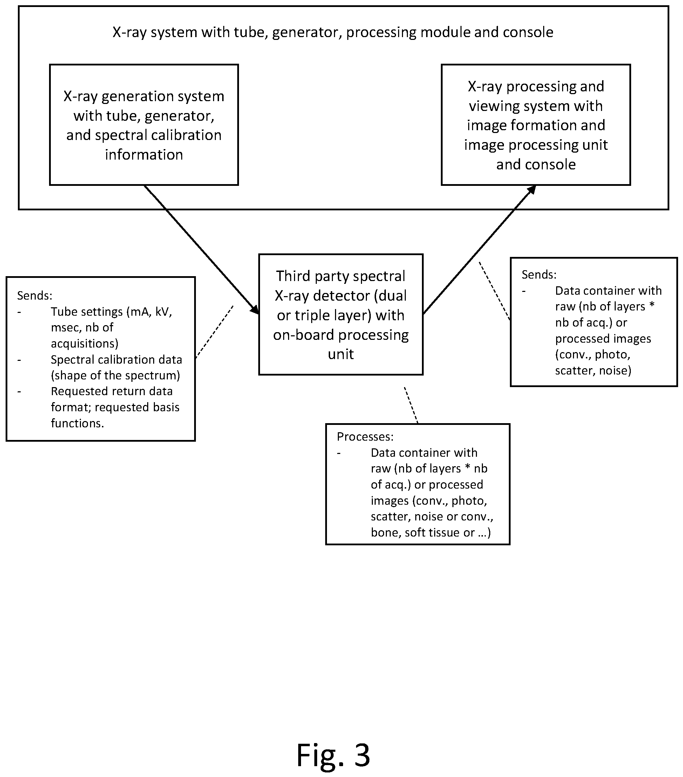

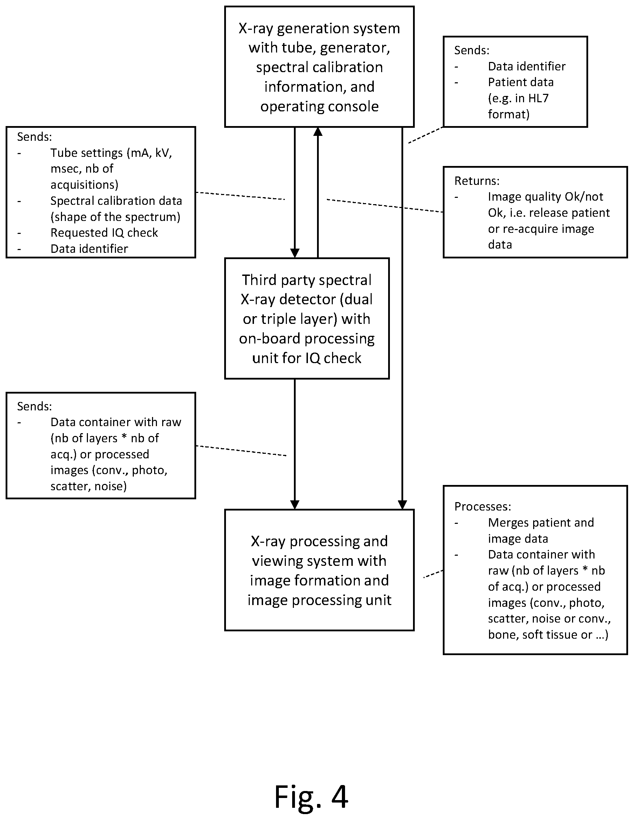

Figs. 3 and 4 show block diagrams with data flow and interfaces of a computer-implemented method for converting image data of an X-ray detector to a predefined data format of an X-ray control device according to embodiments of the invention. Exemplary embodiments can be a spectral detector with integrated pre-processing, where the spectral detector could be a dual layer, a triple layer, or a photon counting detector, or a spectral detector with no pre-processing and converting/processing interface. Dual kV imaging with multiple images at different kV settings can be performed for one combined medical image. A system with multiple, may be wireless, detectors for imaging of different angular views or with different sizes could be utilized. A cloud connection from the detector to the cloud and from the X-ray system to the cloud in cases that the detector itself does not carry a processing unit while the X-ray system expects processed spectral images can be provided, while the image processing is performed in the cloud. DAX detector with an interface for preprocessing and system synchronization can be provided. An x-ray system with tube, generator, and operating console only for imaging settings and performing measurements and patient information; system can create an anonymized but patient specific identifier and send patient information directly to X-ray processing and viewing system. A spectral detector with integrated data assessment or pre-processing allows to initially check image quality in a subset of acquired data, e.g., correct field-of-view, no motion artifacts etc. Image quality IQ can be assessed, e.g., with specialized neural networks or other image analysis algorithms, or the data can be linked to a patient specific identifier. A spectral detector can provide result of image quality assessment to the operator of the imaging system to release the patient, or complete image formation can be performed on the X-ray processing and viewing system, and image data can be merged with patient information, e.g., via a patient identifier. For simultaneous use of detectors from different vendors in a multi-layer system, the data interface is proposed to contain, optionally spectrally resolved, calibration data for absorption and scattering in more proximal layers. Proximal detectors may also be retrofitted with absorption markers that allow exact lateral alignment of the use of calibration data when correcting images of more distal detectors.

Thus, when diagnostic X-ray imaging is extended towards spectral imaging, interfaces to spectral X-ray detectors will enable an existing imaging system to generalize to these concepts. This will include detecting which detector is in use to adapt acquisition settings and to prepare the processing and display chain. Detector output can be transferred via standard data container as, for example, used in spectral CT - a type of 2D SBI comprising a photo image, a scatter image, a conventional image, and a noise image.

The detection of the type of detector can be done in different ways starting with known detectors and known data transmission parameters often having an initialization data block at the start of the system which is used for identification. A second option is the configuration of the interface with entering the data format information selecting the detector from a list of supported devices. In case no exact detector information is available a search-and-define-run could be started to initiate sending of data by typical data signals known from the vendor and doing a detailed data analysis of the sent data. A known phantom may help to analyze the typical image in a spectral scan to analyze the image size and the sequence of data that has to represent exactly what has been scanned as a phantom.

The proposed method can also be extended to systems enabling dual energy imaging via the tube or dark field X-ray images as sequence of defined images in a defined sequence and linked to defined system settings.

The proposed method and the corresponding interface can be utilized in combination with a DXR detector interface, but maybe also relevant for image guided therapy. It can applicable to dual layer, triple layer, and photon counting detectors as well as phase contrast imaging.

With the method according to the invention, it is possible to map special additional data information of specific detectors into a standard data container expected by an X-ray imaging system, which allows to make the imaging system upgrade-ready for the future. The standard data format allows to use spectral diagnostic X-ray data in the processing and display chain of the imaging system, thereby providing a raw-to-imaging-system data compatibility. The data conversion may also happen by the data receiver being able to convert multiple different data formats of different vendors.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. The mere fact that certain measures are re-cited in mutually different dependent claims does not indicate that a combination of these measures cannot be used to advantage. Any reference signs in the claims should not be construed as limiting the scope.

It is to be understood that any of the previous steps described in relation to embodiments and/or training steps described above can be performed by a specific-purpose computer system or general-purpose computer system, or a computer-readable medium, or data carrier system configured to carry out any of the steps described previously. The computer system can include a set of software instructions that can be executed to cause the computer system to perform any of the methods or computer-based functions disclosed herein. The computer system may operate as a standalone device or may be connected, for example using a network, to other computer systems or peripheral devices. In embodiments, a computer system performs logical processing based on digital signals received via an analogue-to-digital converter.

However, all of these and similar terms are to be associated with the appropriate physical quantities and are merely convenient labels applied to these quantities. Unless specifically stated otherwise as apparent from the following discussion, it is appreciated that throughout the description, discussions utilizing terms such as "processing" or "computing" or "calculating" or "determining" or "displaying" or the like, refer to the action and processes of a computer system, or similar electronic computing device, that manipulates and transforms data represented as physical (electronic) quantities within the computer system memories or registers or other such information storage devices. Portions of the present disclosure include processes and instructions that may be embodied in software, firmware, or hardware, and when embodied in software, may be downloaded to reside on and be operated from different platforms used by a variety of operating systems.

In a networked deployment, the computer system operates in the capacity of a server, or as a client user computer in a server-client user network environment, or as a peer computer system in a peer-to-peer or distributed network environment. The computer system can also be implemented as or incorporated into various devices, such as a server or another type of computer such as a workstation that includes a controller, a stationary computer, a mobile computer, a personal computer (PC), a laptop computer, a tablet computer, or any other machine capable of executing a set of software instructions sequentially or non-sequentially that specify actions to be taken by that machine. The computer system can be incorporated as an integrated system part of a larger system that includes additional devices. In an embodiment, the computer system can be implemented using electronic devices that provide voice, video, or data communication possibilities. Further, while the computer system is illustrated in the singular, the term "system" shall also be taken to include any collection of systems or sub-systems that individually or jointly execute a set or multiple sets, of software instructions to perform one or more computer functions.

The computer system may also include a processor. The processor executes instructions to implement some, or all aspects of methods and processes described herein. The processor is tangible and non-transitory. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The processor is an article of manufacture and/or a machine component. The processor is configured to execute software instructions to perform functions as described in the various embodiments herein. The processor may be a general-purpose processor or may be part of an application specific integrated circuit (ASIC). The processor may also be a microprocessor, a microcomputer, a processor chip, a controller, a microcontroller, a digital signal processor (DSP), a state machine, or a programmable logic device, a logical circuit, including a programmable gate array (PGA), such as a field programmable gate array (FPGA), or another type of circuit that includes discrete gate and/or transistor logic. The processor may be a central processing unit (CPU), a graphics processing unit (GPU), or both. Additionally, any processor described herein may include multiple processors, parallel processors, or both. Multiple processors may be included in, or coupled to, a single device or multiple devices. The processor can include one or more internal levels of cache, and a bus controller or bus interface unit to direct interaction with a bus. The term "processor" as used herein encompasses an electronic component able to execute a program or machine executable instruction. References to a computing device comprising "a processor" should be interpreted to include more than one processor or processing core, as in a multi-core processor. A processor may also refer to a collection of processors within a single computer system or distributed among multiple computer systems. The term computing device should also be interpreted to include a collection, or network, of computing devices each including a processor or processors. Programs have software instructions performed by one or multiple processors that may be within the same computing device or which may be distributed across multiple computing devices. Further, the software instructions, when executed by the processor, perform one or more steps of the methods and processes as described herein.

The computer system can further include a communications interface by way of which the computer system can connect to networks and receive data useful in executing the methods and system set out herein as well as transmitting information to other devices. The computer system further includes a video display unit as an output device by which information can be output, such as a liquid crystal display (LCD), an organic light emitting diode (OLED), a flat panel display, a solid-state display, or a cathode ray tube (CRT), for example. Additionally, the computer system includes an input device, such as a keyboard/virtual keyboard or touch-sensitive input screen or speech input with speech recognition, and a cursor control device, such as a mouse or touch-sensitive input screen or pad. The computer system also optionally includes a disk drive unit, a signal generation device, such as a speaker or remote control, and/or a network interface device.

The processes and displays presented herein are not inherently related to any particular computer or other apparatus. Various general-purpose systems may also be used with programs in accordance with the teachings herein, or it may prove convenient to construct more specialized apparatus to perform one or more method steps. The structure for a variety of these systems is discussed in the description below. In addition, any programming language that is sufficient for achieving the techniques and implementations of the present disclosure may be used. In addition, the language used in the specification has been principally selected for readability and instructional purposes and may not have been selected to delineate or circumscribe the disclosed subject matter. Accordingly, the present disclosure is intended to be illustrative, and not limiting, of the scope of the concepts discussed herein.

In accordance with various embodiments of the present disclosure, the methods described herein may be implemented using a hardware computer system that executes software programs. Further, in an exemplary, non-limited embodiment, implementations can include distributed processing, component/object distributed processing, and parallel processing. Virtual computer system processing may implement one or more of the methods or functionalities as described herein, and a processor described herein may be used to support a virtual processing environment.

The illustrations of the embodiments described herein are intended to provide a general understanding of the structure of the various embodiments. The illustrations are not intended to fully describe all the elements and features of the disclosure described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

### LIST OF REFERENCE SIGNS:

- 100: X-ray imaging system
- 110: processing unit
- 120: interface unit
- 121: first interface
- 122: second interface
- 130: X-ray control device
- 140: X-ray detector
- 150: X-ray image data
- 160: processed X-ray image
- 170: request
- 180: trigger signal

## Claims

1. A computer-implemented method for converting image data of an X-ray detector (140) to a predefined data format of an X-ray control device (130), the method comprising:
receiving (S110) a request (170) from the X-ray control device (130) to provide an X-ray image, the request (170) comprising a set of predefined image parameters of the X-ray image;
sending (S120) a trigger signal (180) to the X-ray detector (140) to provide X-ray image data (150) acquired with the X-ray detector;
receiving (S130) the X-ray image data (150) acquired by the X-ray detector from the X-ray detector (140);
processing (S140) the received X-ray image data (150), thereby generating a processed X-ray image (160) comprising the predefined image parameters; and
providing (S150) the processed X-ray image (160) to the X-ray control device (130).

2. The method according to claim 1, wherein the set of predefined image parameters comprises a predefined size of the X-ray image, and wherein processing the received X-ray image data (150) comprises interpolating the received X-ray image data (150) to generate the processed X-ray image (160) with the predefined size.

3. The method according to any one of the preceding claims, further comprising determining a data format of the received X-ray image data (150), and wherein processing the received X-ray image data (150) comprises translating the determined data format of the received X-ray image data (150) into a predefined data format.

4. The method according to claim 3, wherein determining a data format of the received X-ray image data (150) comprises
comparing the received X-ray image data (150) with a plurality of data formats from a database;
receiving information indicating the data format of the received X-ray image data (150) from the X-ray detector (140) or from an operator of the X-ray control device (130); and/or using a trained artificial intelligence to determine the data format of the received X-ray image data (150).

5. The method according to any one of the preceding claims, further comprising analyzing the received X-ray image data (150) with respect to an indication of whether the received X-ray image data (150) has been acquired by the X-ray detector (140) using an anti-scatter grid; and
in case an indication of an anti-scatter is detected, processing the received X-ray image data (150) comprises the removing grid artefacts of the anti-scatter grid from the received X-ray image data (150), or
in case an indication of no anti-scatter is detected, processing the received X-ray image data (150) comprises performing scatter correction of the received X-ray image data (150).

6. The method according to any one of the preceding claims, further comprising
receiving imaging parameters of the X-ray control device (130); and wherein processing the received X-ray image data (150) comprises processing the received X-ray image data (150) on the basis of the received imaging parameters of the X-ray control device (130).

7. The method according to claim 6, wherein the imaging parameters comprise an energy setting and/or an energy spectrum of an X-ray tube connected to the X-ray control device (130).

8. The method according to any one of claims 1 to 7, wherein the X-ray detector (140) is a spectral detector comprising a plurality of detection layers, wherein the received X-ray image data (150) comprises a plurality of sub-images corresponding to the plurality of detection layers, and wherein processing the received X-ray image data (150) comprises at least one of
correcting at least one of the plurality of sub-images for absorption effects due to another one of the plurality of sub-images,
performing spectral processing using an X-ray energy spectrum of an X-ray tube connected to the X-ray control device (130), and
calculating a conventional X-ray image based on the plurality of sub-images.

9. The method according to any one of claims 1 to 7, wherein the X-ray detector (140) is a flat panel detector configured to perform phase stepping, wherein the received X-ray image data (150) comprises a plurality of phase-stepping images, and wherein processing the received X-ray image data (150) comprises at least one of
performing phase contrast processing using an X-ray energy spectrum of an X-ray tube connected to the X-ray control device (130) and grid information provided by the X-ray control device (130), and
calculating a conventional X-ray image based on the plurality of phase-stepping images.

10. The method according to any one of the preceding claims, wherein the received X-ray image data (150) comprises a plurality of temporal sub-images acquired by the X-ray detector (140) in a temporal series, and wherein processing the received X-ray image data (150) comprises at least one of
selecting at least one of the plurality of temporal sub-images,
calculating an integral image from the plurality of temporal sub-images,
calculating a registered integral image from the plurality of temporal sub-images, and
selecting a sharpest image from the plurality of temporal sub-images.

11. A data processing apparatus (110) for carrying out the steps of the method according to any one of claims 1 to 10.

12. An interface unit (120) comprising the data processing apparatus (110) of claim 11, the interface unit (120) comprising:
a first interface (121) configured to be communicationally connected to the X-ray control device (130); and
a second interface (122) configured to be communicationally connected to the X-ray detector (130).

13. An X-ray imaging system (100) comprising:
the interface unit (120) of claim 12;
an X-ray control device (130); and
an X-ray detector (140).

14. A computer program comprising instructions which, when the program is executed by a computer, cause the computer to carry out the steps of the method according to any one of claims 1 to 10.

15. A computer-readable storage medium comprising instructions which, when executed by a computer, cause the computer to carry out the steps of the method according to any of claims 1 to 10.
